# EUROPÄISCHE PATENTSCHRIFT

(11) **EP 3 263 549 B1**
(45) Veröffentlichungstag und Bekanntmachung des Hinweises auf die Patenterteilung: **31.07.2019**
(21) Anmeldenummer: 17000968.2
(22) Anmeldetag: 08.06.2017
(51) Int. Cl.: C07C 209/60, C07C 211/24

(54) **VERFAHREN ZUR SYNTHESE VON 1,1-DIAMINO-2,2-DINITROETHYLEN (FOX-7) ODER EINES SALZES DAVON**
PROCESS FOR THE PREPARATION OF 1,1-DIAMINO-2,2-DINITROETHYLENE (FOX-7) OR A SALT THEREOF
PROCÉDÉ DE PRÉPARATION DE 1,1-DIAMINO-2,2-DINITROÉTHYLÈNE (FOX-7) OU D'UN SEL DE CELUI-CI

(30) Priorität: 29.06.2016 DE 102016007865
(43) Veröffentlichungstag der Anmeldung: 03.01.2018
(73) Patentinhaber: Diehl Defence GmbH & Co. KG, 88662 ÜBERLINGEN (DE)
(72) Erfinder: Pham-Schönwetter, Oliver, 91207 Lauf (DE); Hahma, Arno, 91239 Henfenfeld (DE); Donner, Björn, 91325 Adelsdorf (DE)
(74) Vertreter: Diehl Patentabteilung

(56) Entgegenhaltungen:
- WO-A1-99/03818
- US-A1- 2012 178 968

## Beschreibung

Die Erfindung betrifft die Synthese von 1,1-Diamino-2,2-dinitroethylen (FOX-7) oder eines Salzes davon. Zur Herstellung von FOX-7 sind verschiedene Methoden bekannt:

### 1. Nitrierung eines Stickstoffheterozyklus mit anschließender Hydrolyse zum Endprodukt

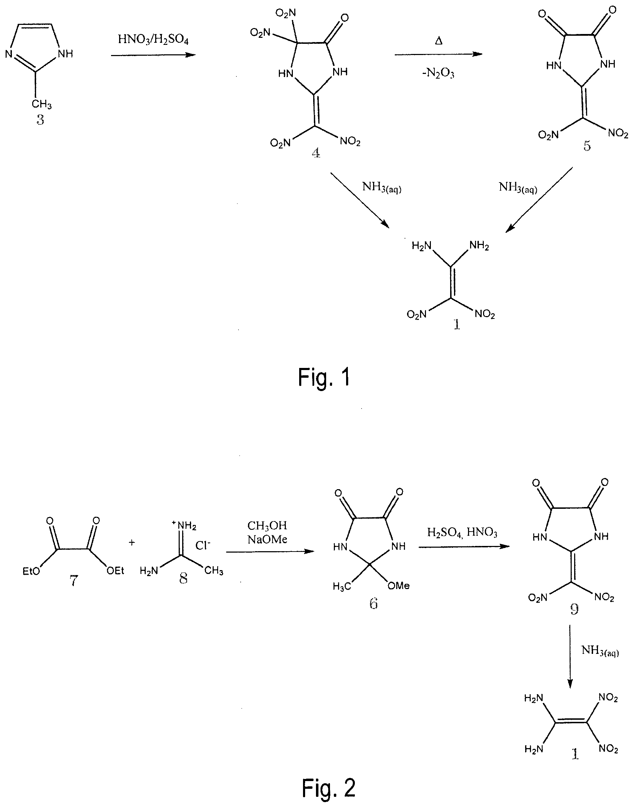

Die Synthese ist in Fig. 1 schematisch dargestellt. Bei dem Stickstoffheterozyklus kann es sich beispielsweise um Methylimidazol (3) handeln. Bei dessen Nitrierung mittels HNO₃/H₂SO₄ kommt es durch eine Nitrierung der Methylgruppe und einer Oxidation der beiden nicht an der Bindung der Methylgruppe beteiligten Kohlenstoffatome des Imidazolrings zur Bildung einer Zwischenverbindung (4). Diese Zwischenverbindung (4) zerfällt bei Raumtemperatur teilweise in eine weitere Zwischenverbindung (5), wobei aus beiden Zwischenverbindungen (4, 5) durch Hydrolyse in flüssigem Ammoniak FOX-7 (1) entsteht. Problematisch bei dieser Synthese ist, dass die weitere Zwischenverbindung (5) sehr empfindlich ist und leicht explodiert. Weiterhin ist die Nitrierungsreaktion verhältnismäßig ineffizient. Dies führt zu einer geringen Gesamtausbeute von üblicherweise höchstens 13%.

### 2. Nitrierung von 2-Methyl-2-Methoxyimidazoldion (6)

Die Reaktion ist in Fig. 2 dargestellt. Das 2-Methyl-2-Methoxyimidazoldion (6) kann durch Kondensation der Verbindung (7) mit Acetamidinhydrochlorid (8) in Gegenwart von Natriummethoxid in Methanol hergestellt werden. Die Nitrierung von 2-Methyl-2-Methoxyimidazoldion (6) erlaubt üblicherweise eine Gesamtausbeute von bis zu 38%.

Nachteilig bei der Methode ist, dass das entstehende Zwischenprodukt (9) empfindlich ist und leicht explodiert und dass bei der Reaktion Reaktionswärme entsteht. Daher muss aus Sicherheitsgründen in großer Verdünnung gearbeitet werden. Dadurch sind verhältnismäßig große Reaktionsgefäße erforderlich, wodurch eine großtechnische Synthese unwirtschaftlich ist.

### 3. Nitrierung von 2-Methyl-4,6-pyrimidindion (10)

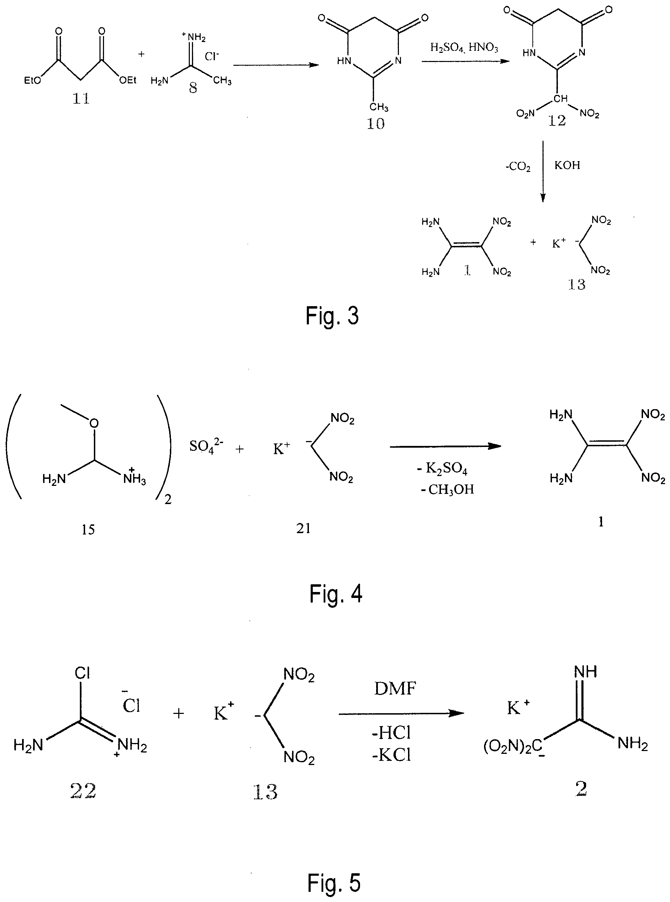

Das Verfahren ist in Fig. 3 dargestellt. 2-Methyl-4,6-pyrimidindion kann aus Diethylmalonat (11) und Acetamidinhydrochlorid (8) hergestellt werden. Die Ausbeute beträgt etwa 80%. Bei der Hydrolyse der nitrierten Zwischenverbindung (12) entsteht als Nebenprodukt Kaliumdinitromethanat (13). Da Kaliumdinitromethanat explosiv und temperaturempfindlich ist, ist es erforderlich, die Nitrierung langsam und temperaturkontrolliert durchzuführen. Dadurch ist die großtechnische Durchführung der Reaktion verhältnismäßig teuer. Nach Abschluss der Nitrierung wird das Kaliumdinitromethanat (13) durch Filtration abgetrennt.

Weitere Verfahren zur Herstellung von FOX-7 sind aus der WO 99/03818 und der US 2012/0178968 A1 bekannt.

Da FOX-7 bisher verhältnismäßig teuer ist, findet es außer als Boosterladung für nukleare Gefechtsköpfe, Zündüberträger und Zündverstärker in Kleinmengen bisher kaum industrielle Anwendung. Wegen seiner hohen Leistung und geringen Sensitivität gegenüber Schlag, Reibung und thermischen Einflüssen wäre FOX-7 als Hochleistungssprengstoff für viele Anwendungen attraktiv, wenn es günstiger herzustellen wäre. Beispielsweise könnte es als insensitiver Leistungsverstärker in Scheinzielsätzen für spektrale Flares, als Leistungsverstärker in Treibsätzen sowie als Hauptladung in diversen Anwendungen eingesetzt werden. Aufgabe der vorliegenden Erfindung ist es daher, ein alternatives Verfahren zur günstigen Herstellung von FOX-7 bereitzustellen.

Die Aufgabe wird durch die Merkmale des Patentanspruchs 1 gelöst. Zweckmäßige Ausgestaltungen ergeben sich aus den Merkmalen der Patentansprüche 2 bis 9.

Erfindungsgemäß ist ein Verfahren zur Synthese von 1,1-Diamino-2,2-dinitroethylen (FOX-7) oder eines Salzes davon vorgesehen, wobei ein Isoharnstoffderivat-Kation mit einem Dinitromethan-Anion umgesetzt wird. Das Isoharnstoffderivat-Kation besteht dabei aus einem C(NH₂)(NH₂⁺)-Rest und einer an das C-Atom dieses C(NH₂)(NH₂⁺)-Rests gebundenen nucleofugen Abgangsgruppe, welche wie im Anspruch 1 spezifiziert ist. Das Umsetzen des Isoharnstoffderivat-Kations mit dem Dinitromethan-Anion erfolgt in einer Lösung. Es kann insbesondere in einem polaren aprotischen Lösungsmittel, wie beispielsweise Dimethylformamid (DMF), erfolgen.

Bei der Reaktion erfolgt eine nucleophile Substitution der Abgangsgruppe durch das Dinitromethan-Anion an dem Isoharnstoffderivat-Kation. Durch die an dem C(NH₂)(NH₂⁺)-Rest gebundene nucleophile Abgangsgruppe ist das Isoharnstoffderivat aktiviert. Dadurch, dass die Abgangsgruppe nucleofug ist, ist das Isoharnstoffderivat bei der nucleophilen Substitutionsreaktion das Elektrophil.

Das erfindungsgemäße Verfahren ist wesentlich kostengünstiger durchzuführen als die im Stand der Technik bekannten Synthesen. Dies liegt unter anderem daran, dass das Verfahren wesentlich ungefährlicher durchzuführen ist, da dabei kein gefährlicher Nitrierungsschritt erfolgt und die Reaktion bei einer verhältnismäßig niedrigen Temperatur in Lösung abläuft. Das Produkt kann aus der Lösung ausgefällt und abfiltriert werden. Bei der Reaktion entsteht primär ein Salz von FOX-7, welches sich durch Ansäuern oder Neutralisieren der oder einer das Salz enthaltenden Lösung in FOX-7 überführen lässt. Da die Synthese bei einer verhältnismäßig niedrigen Temperatur abläuft, stellt auch der Einsatz des an sich empfindlichen und explosiven Dinitromethan-Anions kein Sicherheitsproblem dar.

Bei der nucleofugen Abgangsgruppe handelt es sich um einen O-Alkyl-, insbesondere O-Methyl-, O-Ethyl-, O-Propyl-, O-Butyl- oder O-Pentyl-, Triazol-, Halogenid-, insbesondere Chlorid-, Carbonsäurehalogenid-, insbesondere Carbonsäurechlorid-, Nonaflat-, Trifluormethansulfat-, Sulfonat-, insbesondere Halogensulfonat-, insbesondere Fluorsulfonat-, Tosyl-, Mesyl-, Diazonium-, Oxonium-, quartären Ammoniumverbindungs-, Ester-, Säureanhydrid-, Nitrat-, Phosphat-, organischen Ester-, Ammonium-, Phenol-, Alkohol- oder einen Carbonsäure-Rest. Die O-alkylierten Isoharnstoffderivate besitzen durch die Alkylierung am Carbonylsauerstoff des Harnstoffs eine hohe Reaktivität, da die O-Alkylgruppe eine leicht abspaltbare Abgangsgruppe ist.

Eine alternative Abgangsgruppe stellt ein Halogenid, beispielsweise ein Chlorid, dar. Beispielsweise kann das Isoharnstoffderivat-Kation als Chlorformamidinium-hydrochlorid vorliegen, welches beispielsweise durch wasserfreie Hydrochlorierung von Cyanamid hergestellt werden kann. Dabei entsteht ein Hydrochlorid-Addukt mit einer Chlor-Abgangsgruppe am Amidinium-Kohlenstoff.

Eine weitere, gut geeignete Abgangsgruppe ist ein Triazol-Rest. Ein Isoharnstoffderivat-Kation mit dieser Abgangsgruppe liegt beispielsweise im kommerziell verfügbaren Triazolcarboxamidinhydrochlorid vor.

Bei dem Isoharnstoffderivat-Kation kann es sich demnach um ein Chlorformamidinium-Ion oder ein Triazolcarboxamidin-Ion handeln. Das Chlorformamidinium-Ion oder das Triazolcarboxamidin-Ion kann mit Chlorid als Gegenion assoziiert sein. Grundsätzlich kann das Isoharnstoffderivat-Kation mit einem Sulfat, Hydrogensulfat, Acetat oder Halogenid, insbesondere Chlorid, als Gegenion assoziiert sein.

Das Dinitromethan-Anion kann mit einem Kalium-Ion oder Ammonium-Ion als Gegenion assoziiert sein.

Ein bei der Synthese entstehendes 1,1-Diamino-2,2-dinitroethylen-Anion kann durch Verminderung des pH-Werts der/einer das 1,1-Diamino-2,2-dinitroethylen-Anion enthaltenden Lösung in 1,1-Diamino-2,2-dinitroethylen überführt werden. Bei der Lösung kann es sich um die Lösung handeln, in welcher die Synthese erfolgt ist. Es ist jedoch auch möglich, dass das 1,1-Diamino-2,2-dinitroethylen-Anion in Form eines Salzes aus der Lösung, in welcher die Synthese erfolgt ist, abgesondert und dann im selben oder einem anderen Lösungsmittel gelöst wird, um die genannte Lösung zu bilden.

Nachfolgend wird die Erfindung anhand von Ausführungsbeispielen näher erläutert. Es zeigen:
- Fig. 4: ein Reaktionsschema der Synthese von FOX-7 aus O-alkyliertem Isoharnstoffsalz,
- Fig. 5: ein Reaktionsschema der Synthese von KFOX-7 aus Chlorformamidiniumhydrochlorid und
- Fig. 6: ein Reaktionsschema der Synthese von KFOX-7 aus Triazolcarboxamidinhydrochlorid.

Zur Synthese von FOX-7 aus O-alkyliertem Isoharnstoffsalz gemäß Fig. 4 werden in einem 100 ml Rundkolben unter Rühren nacheinander 490 mg (3,4 mmol) Kaliumdinitromethanat und 419 mg (3,4 mmol) O-Methylisoharnstoffsulfat in 50 ml Wasser bei 70 °C gelöst. Anschließend wird die Lösung bei 70 °C eine Stunde lang weitergerührt. Der dabei gebildete weiße Niederschlag wird durch Filtration abgesondert. Von der verbleibenden Lösung wird das Lösungsmittel im Rotationsverdampfer langsam abdestilliert. Das Produkt FOX-7 fällt dabei als gelber Niederschlag aus. Zur weiteren Reinigung wird das Produkt in Acetonitril/Methanol 30/70 gelöst und über eine 20 cm lange Silikasäule mit Acetonitril/Methanol 30/70 als Laufmittelgemisch einer Chromatographie unterworfen. Von der das Produkt enthaltenden Fraktion wird das Lösungsmittel mittels eines Rotationsverdampfers abdestilliert.

Ein 1H-NMR-Spektrum, ein 13C-NMR-Spektrum und ein Massenspektrum des Reaktionsprodukts und einer KFOX-7-Referenz zeigen, dass tatsächlich FOX-7 bei der Reaktion entstanden ist. Die Ergebnisse der Analytik des Reaktionsprodukts im Vergleich zu der KFOX-7-Referenz sind in der nachfolgenden Tabelle 1 dargestellt:

**Tabelle 1**

| Analytik | Reaktionsprodukt | KFOX-7-Referenz |
|---|---|---|
| 1H-NMR (400 MHz) | 8,4 ppm (s) breit | 8,8 ppm (s) breit (entspricht FOX-7) |
| 13C-NMR (400 MHz) | 158,43 ppm Singulett | 158,41 ppm Singulett |
| Massenspektrum (ESI) | 149 m/z | 149 m/z |

Zur Synthese von KFOX-7 gemäß dem Reaktionsschema gemäß Fig. 5 werden in einem 50 ml Becherglas 73 mg (0,5 mmol) Kaliumdinitromethanat in 20 ml DMF bei 60 °C gelöst und 58 mg (0,5 mmol) Chlorformamidinium-Hydrochlorid zugegeben. Die Lösung wurde 2 Stunden bei 60 °C gerührt und anschießend unter Rühren auf Raumtemperatur abkühlen gelassen. Nach Abdestillieren des Lösungsmittels wurde der Rückstand bei 50 °C in einem explosionsgeschützten Trockenofen getrocknet und im Anschluss thermoanalytisch mittels dynamischer Differenzkalorimetrie (Differential Scanning Calorimetrie, DSC) sowie IR-, NMR- und Massenspektroskopisch vermessen. Das Ergebnis ist in der nachfolgenden Tabelle 2 dargestellt:

**Tabelle 2**

| Analytik | Reaktionsprodukt | K-FOX-7-Referenz |
|---|---|---|
| 1-H NMR (400 Mhz) | 8,54 ppm (s) | 8,88 ppm (s) (entspricht FOX-7) |
| 13-C NMR (400 Mhz) | 158,4 ppm | 158,4 ppm (KFOX-7) |
| Massenspektrum | 149 m/z | 148 m/z |
| | (=M+1-Peak FOX-7) | |
| DSC | 230°C | 220 - 250°C (FOX-7) |

Der Vergleich mit der KFOX-7-Referenz zeigt, dass das Reaktionsprodukt KFOX-7 enthält.

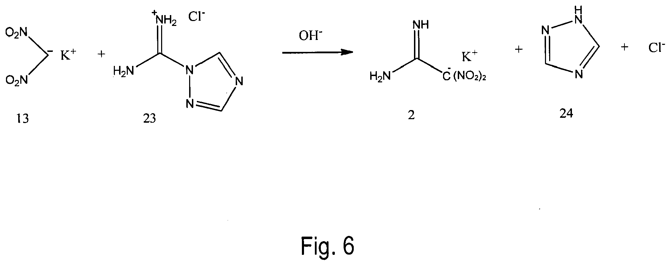

Zur Synthese von KFOX-7 gemäß dem in Fig. 6 dargestellten Reaktionsschema gemäß werden in einem 100 ml Rundkolben 980 mg Kaliumdinitromethanat (6,8 mmol) in 10 ml destilliertem Wasser aufgelöst. Dazu wird 1g 1H-1,2,4-Triazol-1-Carboxamidinhydrochlorid (6,8 mmol) gegeben. Unmittelbar nach der Zugabe werden der pH-Wert mittels 20%iger KOH-Lösung auf pH 11 bis 12 eingestellt und die Temperatur auf 50 °C erhöht. Die Lösung wird über Nacht bei 50 °C gerührt. Anschließend wird das Lösungsmittel im Rotationsverdampfer abdestilliert. Es verbleibt ein gelber Rückstand, der in einer Filterfritte dreimal mit je 20 ml Isopropanol zur Entfernung von Triazol und KOH gewaschen wird. Eine Bestimmung der Zersetzungstemperatur mittels DSC ergibt einen Wert von 222 °C, der mit dem Wert der KFOX-7-Referenz übereinstimmt. Ein 13C-NMR-Spektrum zeigt für KFOX-7 einen Peak, der identisch mit dem KFOX-7 Peak einer Referenzprobe ist. Auch eine massenspektroskopische Untersuchung zeigt eine Übereinstimmung des Reaktionsprodukts mit der KFOX-7-Referenz. Die Ergebnisse sind in der nachfolgenden Tabelle 3 dargestellt.

**Tabelle 3**

| Analytik | Reaktionsprodukt | KFOX-7-Referenz |
|---|---|---|
| 13C-NMR | 158,4 ppm Singulett | 158,41 ppm Singulett |
| Massenspektrum | 149 m/z | 149 m/z |
| DSC | 228 °C | 228 °C |

Der Vergleich mit der KFOX-7-Referenz zeigt, dass das Reaktionsprodukt KFOX-7 enthält.

Bei den Ausführungsbeispielen kann statt des Kaliumdinitromethanats auch Ammoniumdinitromethanat eingesetzt werden.

## Patentansprüche

1. Verfahren zur Synthese von 1,1-Diamino-2,2-dinitroethylen (FOX-7) oder eines Salzes davon,
**dadurch gekennzeichnet,**
**dass** ein Isoharnstoffderivat-Kation, welches aus einem C(NH₂)(NH₂⁺)-Rest und einer an das C-Atom dieses C(NH₂) (NH₂⁺)-Rests gebundenen nucleofugen Abgangsgruppe besteht, mit einem Dinitromethan-Anion umgesetzt wird, wobei die nucleofuge Abgangsgruppe aus einer Gruppe ausgewählt ist, die aus einem O-Alkyl-, Triazol-, Halogenid-, Carbonsäurehalogenid-, Nonaflat-, Trifluormethansulfat-, Sulfonat-, Tosyl-, Mesyl-, Diazonium-, Oxonium-, quartären Ammoniumverbindungs-, Ester-, Säureanhydrid-, Nitrat-, Phosphat-, organischen Ester-, Ammonium-, Phenol-, Alkohol- und einem Carbonsäure-Rest besteht.

2. Verfahren nach Anspruch 1,
**dadurch gekennzeichnet,**
**dass** das Umsetzen des Isoharnstoffderivat-Kations mit dem Dinitromethan-Anion in einem polaren aprotischen Lösungsmittel erfolgt.

3. Verfahren nach Anspruch 2,
**dadurch gekennzeichnet,**
**dass** das Lösungsmittel Dimethylformamid (DMF) ist.

4. Verfahren nach einem der vorhergehenden Ansprüche,
**dadurch gekennzeichnet,**
**dass** der O-Alkyl-Rest ein O-Methyl-, O-Ethyl-, O-Propyl-, O-Butyl- oder O-Pentyl-Rest, der Halogenid-Rest ein Chlorid-Rest, der Carbonsäurehalogenid-Rest ein Carbonsäurechlorid-Rest und der Sulfonat-Rest ein Halogensulfonat-Rest, insbesondere ein Fluorsulfonat-Rest ist.

5. Verfahren nach einem der vorhergehenden Ansprüche,
**dadurch gekennzeichnet,**
**dass** das Isoharnstoffderivat-Kation Chlorformamidinium-Ion oder Triazolcarboxamidin-Ion ist.

6. Verfahren nach einem der vorhergehenden Ansprüche,
**dadurch gekennzeichnet,**
**dass** das Isoharnstoffderivat-Kation mit einem Sulfat, Hydrogensulfat, Acetat oder Halogenid, insbesondere Chlorid, als Gegenion assoziiert ist.

7. Verfahren nach Anspruch 5,
**dadurch gekennzeichnet,**
**dass** das Isoharnstoffderivat-Kation mit Chlorid als Gegenion assoziiert ist.

8. Verfahren nach einem der vorhergehenden Ansprüche,
**dadurch gekennzeichnet,**
**dass** das Dinitromethan-Anion mit einem Kalium-Ion oder Ammonium-Ion als Gegenion assoziiert ist.

9. Verfahren nach einem der vorhergehenden Ansprüche,
**dadurch gekennzeichnet,**
**dass** ein bei der Synthese entstehendes 1,1-Diamino-2,2-dinitroethylen-Anion durch Verminderung des pH-Werts einer das 1,1-Diamino-2,2-dinitroethylen-Anion enthaltenden Lösung in 1,1-Diamino-2,2-dinitroethylen überführt wird.

## Claims

1. Method for synthesizing 1,1-diamino-2,2-dinitroethylene (FOX-7) or a salt thereof,
**characterized in that**
an isourea derivative cation, which consists of a C(NH₂) (NH₂⁺) radical and a nucleofugal leaving group bonded to the carbon atom of said C(NH₂) (NH₂⁺) radical, is reacted with a dinitromethane anion, wherein the nucleofugal leaving group is selected from a group consisting of an O-alkyl, triazolyl, halide, carboyl halide, nonaflate, trifluoromethanesulphate, sulphonate, tosyl, mesyl, diazonium, oxonium, quaternary ammonium compound, ester, acid anhydride, nitrate, phosphate, organic ester, ammonium, phenol, alcohol and a carboxylic radical.

2. Method according to Claim 1,
**characterized in that**
the reaction of the isourea derivative cation with the dinitromethane anion takes place in a polar aprotic solvent.

3. Method according to Claim 2,
**characterized in that**
the solvent is dimethylformamide (DMF).

4. Method according to any of the preceding claims,
**characterized in that**
the O-alkyl radical is an O-methyl, O-ethyl, O-propyl, O-butyl or O-pentyl radical, the halide radical is a chloride radical, the carbonyl halide radical is a carbonyl chloride radical and the sulphonate radical is a halosulphonate radical, in particular a fluorosulphonate radical.

5. Method according to any of the preceding claims,
**characterized in that**
the isourea derivative cation is a chloroformamidinium ion or triazolecarboxamidine ion.

6. Method according to any of the preceding claims,
**characterized in that**
the isourea derivative cation is associated with a sulphate, hydrogen sulphate, acetate or halide, particularly chloride, as counterion.

7. Method according to Claim 5,
**characterized in that**
the isourea derivative cation is associated with chloride as counterion.

8. Method according to any of the preceding claims,
**characterized in that**
the dinitromethane anion is associated with a potassium ion or ammonium ion as counterion.

9. Method according to any of the preceding claims,
**characterized in that**
a 1,1-diamino-2,2-dinitroethylene anion formed in the synthesis is converted into 1,1-diamino-2,2-dinitroethylene by lowering the pH of a solution comprising the 1,1-diamino-2,2-dinitroethylene anion.

## Revendications

1. Procédé de synthèse de 1,1-diamino-2,2-dinitroéthylène (FOX-7) ou d'un sel de celui-ci, **caractérisé en ce qu'**un cation d'un dérivé d'isourée, qui est constitué par un radical C(NH₂) (NH₂⁺) et un groupe partant nucléofuge relié à l'atome C de ce radical C(NH₂) (NH₂⁺), est mis en réaction avec un anion dinitrométhane, le groupe partant nucléofuge étant choisi dans un groupe constitué par un radical alkyle, triazole, halogénure, halogénure d'acide carboxylique, nonaflate, trifluorométhane-sulfate, sulfonate, tosyle, mésyle, diazonium, oxonium, composé d'ammonium quaternaire, ester, anhydride d'acide, nitrate, phosphate, ester organique, ammonium, phénol, alcool et acide carboxylique.

2. Procédé selon la revendication 1, **caractérisé en ce que** la réaction du cation d'un dérivé d'isourée avec l'anion dinitrométhane a lieu dans un solvant aprotique polaire.

3. Procédé selon la revendication 2, **caractérisé en ce que** le solvant est le diméthylformamide (DMF).

4. Procédé selon l'une quelconque des revendications précédentes, **caractérisé en ce que** le radical O-alkyle est un radical O-méthyle, O-éthyle, O-propyle, O-butyle ou O-pentyle, le radical halogénure est un radical chlorure, le radical halogénure d'acide carboxylique est un radical chlorure d'acide carboxylique, et le radical sulfonate est un radical halogénosulfonate, notamment un radical fluorosulfonate.

5. Procédé selon l'une quelconque des revendications précédentes, **caractérisé en ce que** le cation d'un dérivé d'isourée est un ion chloroformamidinium ou un ion triazolocarboxamidine.

6. Procédé selon l'une quelconque des revendications précédentes, **caractérisé en ce que** le cation d'un dérivé d'isourée est associé avec un sulfate, un hydrogénosulfate, un acétate ou un halogénure, notamment un chlorure, en tant que contre-ion.

7. Procédé selon la revendication 5, **caractérisé en ce que** le cation d'un dérivé d'isourée est associé avec un chlorure en tant que contre-ion.

8. Procédé selon l'une quelconque des revendications précédentes, **caractérisé en ce que** l'anion dinitrométhane est associé avec un ion potassium ou un ion ammonium en tant que contre-ion.

9. Procédé selon l'une quelconque des revendications précédentes, **caractérisé en ce qu'**un anion 1,1-diamino-2,2-dinitroéthylène formé lors de la synthèse est transformé en 1,1-diamino-2,2-dinitroéthylène par réduction du pH d'une solution contenant l'anion 1,1-diamino-2,2-dinitroéthylène.
